Europäisches Patentamt

European Patent Office

Office européen des brevets

(11) Numéro de publication: **0 134 367**
**A1**

## (12) DEMANDE DE BREVET EUROPEEN

(21) Numéro de dépôt: **83401656.0**

(51) Int. Cl.⁴: **A 61 N 1/04**

(22) Date de dépôt: **12.08.83**

(43) Date de publication de la demande:
20.03.85 Bulletin 85/12

(84) Etats contractants désignés:
BE CH DE FR GB IT LI NL

(71) Demandeur: Alfandari, Jean-Pierre
53 rue Bretonneau
F-37100 Saint Cyr(FR)

(72) Inventeur: Alfandari, Jean-Pierre
53 rue Bretonneau
F-37100 Saint Cyr(FR)

(74) Mandataire: Rataboul, Michel
Cabinet Michel Rataboul 69, rue de Richelieu
F-75002 Paris(FR)

(54) **Dispositif de stimulation cardiaque comprenant une électrode épicardique distincte d'un moyen de fixation.**

(57) Le dispositif de stimulation cardiaque est particulièrement efficient car il permet la pose de deux électrodes, l'une
sur l'oreillette droite et l'autre sur le ventricule droit pour
provoquer des contractions synchrones conformes à la
physiologie.

Ce dispositif est du type comprenant d'une part une
électrode conductrice (1) devant être placée à l'extérieur du
coeur et d'autre part des crochets de fixation (6).

Il est caractérisé en ce que les extrémités libres (6a) des
crochets (6) sont situées au-delà de la partie la plus
proéminente d'une embase (2) et en particulier du sommet
de l'électrode (1) afin qu'ils atteignent directement la paroi
cardiaque, avant que tout autre élément ait pu la repousser.

Pour éviter la protrusion de l'électrode (1) quand on
redresse les crochets (6) par cintrage de l'embase (2), celle-ci
contient un élément rigide tel qu'un organe (8).

FIG. 3

EP 0 134 367 A1

Dispositif de stimulation cardiaque comprenant une électrode
épicardique distincte d'un moyen de fixation

La présente invention concerne un dispositif permettant de réaliser d'une manière particulièrement efficace l' implantation d'une électrode épicardique.

On connait déjà des dispositifs comprenant une électrode épicardique devant pénétrer dans le muscle cardiaque et associée à une embase élastiquement déformable.

Cela présente un inconvénient majeur car la petite blessure subie par le muscle cardiaque lors de la pénétration de l'électrode provoque, comme cela est naturel, une cicatrisation obtenue grâce au fait que le sang serète et accumule de la fibrine sur cette plaie, mais cette fibrine est un mauvais conducteur de l'électricité.

De ce fait la pénétration de l'électrode dans le muscle cardiaque est à l' origine d'une détérioration de la conductivité électrique ce qui est, évidemment, contraire au but recherché, à savoir une conductivité électrique bonne dès l'origine et pour longtemps.

Cette disposition présente toujours les mêmes inconvénients, que le support comprenne des organes de fixation distincts de l'électrode proprement dite ou confondus avec elle.

On connait, ainsi, par exemple une électrode en forme de queue de cochon que l'on fait pénétrer dans le muscle cardiaque à la manière d'une vis.

Cette disposition présente un inconvénient supplémentaire qui est de rendre pratiquement impossible l'implantation d'une telle électrode épicardique ailleurs que sur un ventricule, seul à présenter une paroi assez épaisse pour servir de support acceptable, contrairement aux oreillettes.

Or, étant donné que le sang circule dans le sens de l'oreillette droite vers le ventricule droit, il serait naturel de provoquer la contraction de l'oreillette avant celle du ventricule. Dans la pratique, une électrode épicardique ne pouvant être fixée que sur le ventricule, il peut se produire une contraction rétrograde de l'oreillette qui gêne la circulation et peut occasionner des troubles pulmonaires.

On a déjà pensé à utiliser des stimulateurs cardiaques comprenant deux dispositifs à électrode, l'un placé sur l'oreillette droite et l'autre sur le ventricule droit afin de provoquer des contractions synchrones d'abord de l'oreillette droite puis du ventricule droit.

Malheureusement, ces stimulateurs ne rencontrent pas le succès qu'ils méritent du fait que l'implantation d'un dispositif à électrode épicardique sur l'oreillette est tout à fait aléatoire.

On conçoit en effet qu'il est pratiquement impossible d'obtenir une fixation sérieuse avec une électrode en forme de queue de cochon qui aurait moins d'un millimètre de longueur et que, même si l'on tentait une telle fixation, on ne pourrait pas être assuré que l'extrémité conductrice de l'électrode est bien située au-plus à mi-chair, ce qui est pourtant indispensable au bon fonctionnement de la stimulation, car si l'électrode traverse complètement la paroi, sa partie conductrice est au-delà de cette paroi et il n'y a plus ni contact ni excitation électrique, de sorte que la stimulation est anéantie.

Lorsque l'électrode est distincte des organes de fixation (généralement des crochets), le problème de la conductivité électrique durable n'est pas réglé pour autant. Comme au moment de la mise en place du dipositif l'électrode est presque toujours située en avant des organes de fixation, elle atteint la paroi cardiaque avant eux et doit pénétrer dans cette paroi pour permettre aux organes de fixation de l'atteindre et d'y pénétrer à leur tour.

Des dispositifs de ce type sont décrits notamment dans les brebets FR-A 2.297.641 et FR-A-2.372.636..

Dans le brevet FR-A-2.297.641, il est précisé que pour faire pénétrer l'électrode, on doit soit pratiquer une incision préalable, soit placer sur l'électrode une pointe acérée devant créer elle-même l'incision.

Pour remédier aux inconvénients rappelés ci-dessus, il faudrait que l'électrode soit maintenue au contact de la face extérieure du muscle cardiaque, sans aucune pénétration, car on éviterait ainsi toute blessure.

Pour cela, on peut fixer le dispositif par un adhésif, comme cela est décrit dans le brevet US-A-4.282.886.

Dans la pratique, cette solution est peu utilisée car la fixation du uispositif est précaire et aléatoire.

La fixation du dispositif par pénétration de crochets reste la solution la plus sûre mais jusqu'à ce jour il n'a pas été possible de concilier la sécurité de cette fixation et la qualité de l'excitation électrique par contact de l'électrode contre la face extérieure de la paroi cardiaque.

En effet, pour être très efficaces, les crochets doivent agir comme les griffes d'une pátte d'animal c'est-à-dire que leurs pointes doivent sensiblement se faire face et non pas s'étendre dans des sens opposés, car à l'appui de l'électrode dirigé de l'extérieur vers l'intérieur il est bon de faire correspondre un contre-appui dirigé de l'intérieur vers l'extérieur et celui-ci ne peut être obtenu que par les crochets agissant ensemble pour former une sorte de support, dans une zone opposée et sensiblement périphérique à celle de l'électrode.

Lorsqu'au contraire les crochets "divergent" comme cela est représenté, notamment, sur les figures 4 à 6 du brevet FR 2.297.641, les forces de maintien s'exercent relativement loin de la zone de l'électrode. Cette solution n'est donc envisageable que si l'électrode pénètre dans la paroi et, même, est sollicitée élastiquement vers l'avant par un ressort ou est accrochée par une pointe en forme d'hameçon.

La présente invention prévoit fondamentalement que l'électrode doit rester à l'extérieur de la paroi cardiaque, constamment appuyée contre elle, et comme on vient de l'expliquer, il est meilleur de prévoir des crochets "convergents".

On se heurte alors à une difficulté de mise en place car celle-ci suppose que l'on déforme l'embase élastique pour que les crochets se placent selon une orientation à peu près perpendiculaire à la paroi cardiaque. Mais pour atteindre cette orientation, les crochets doivent effectuer un mouvement de retrait qui a pour corrolaire la protrusion de l'électrode. Lors de la mise en place, l'électrode atteint la Paroi cardiaque avant les crochets et tend à repousser celle-ci, surtout à l'aplomb de l'oreillette car la paroi est ici très fine: 1 à 1,5 millimètre d'épaisseur. En somme, plus l'électrode est avancée et plus la paroi recule par rapport aux crochets de sorte que ceux-ci ne peuvent être convenablement enfoncés.

La présente invention constitue en particulier une solution à ce problème et permet de réaliser un dispositif qui concilie la qualité durable de l'excitation électrique, la sûreté de la fixation et la simplicité de la mise en place.

On peut donc, notamment, obtenir une stimulation de l'oreillette droite et du ventricule droit.

A cette fin, l'invention a pour objet un dispositif de stimulation cardiaque, du type comprenant une électrode conductrice associée à

4    0134367

une embase élastiquement déformable devant être mise en place sur le coeur d'un patient et être fixée à l'extérieur dudit coeur par des crochets, caractérisé en ce que les extrémités libres des crochets sont situées au-delà de la partie la plus proéminente du dispositif et en particulier du sommet de l'électrode selon une distance substantielle lorsqu'ils sont en position de redressement due à la déformation élastique de l'embase.

Selon d'autres caractéristiques de l'invention :

- le dispositif comprend des moyens aptes à s'opposer à la protrusion de l'électrode tout en permettant le redressement des crochets;

- l'embase devant être déformée élastiquement par cintrage, lesdits moyens sont constitués par un élément rigide dans le sens perpendiculaire à celui selon lequel on doit cintrer l'embase lors de sa mise en place;

- ledit élément est une pièce conductrice en forme de T dont la branche est située dans l'embase perpendiculairement au sens selon lequel on doit cintrer celle-ci et dont le jambage constitue l'électrode;

- lesdits moyens sont constitués par un élément substantiellement plan qui est rigide mais pliable en son milieu et qui est traversé d'un trou central par lequel l'électrode est engagée librement, cet élément étant relié aux crochets et à des appuis destinés à recevoir un effort pour le pliage dudit élément et, ipso facto, le cintrage de l'embase;

- l'électrode est munie d'un épaulement disposé sous ledit élément par rapport à l'extrémité libre de ladite électrode et pouvant buter autour du trou central de l'élément;

- l'embase étant réalisée en matériau élastique isolant emprisonnant l'électrode, les crochets et les appuis, une partie de ce matériau d'enrobage située sous l'électrode par rapport à son extrémité libre, forme un coussinet élastique contenant éventuellement un insert;

- les crochets étant mobiles par rapport à l'embase, lesdits moyens sont constitués par deux éléments superposés substantiellement plans, rigides, indéformables et traversés chacun par un trou central par lesquels l'électrode est engagée librement et bute, par un épaulement dont elle est munie, contre l'élément plan le plus éloigné des extrémités libres des crochets, tandis que des écarteurs mobiles sont disposés latéralement en regard d'un espace qui sépare les deux éléments et sont reliés aux crochets et à des appuis destinés à recevoir un effort pour le rapprochement des écarteurs, l'écartement corrélatif des éléments, ainsi que le retrait de l'électrode et le redressement des crochets;

- les écarteurs sont des coins sollicités élastiquement vers leur position d'écartement, notamment au moyen d'un ressort, tandis que les éléments sont sollicités élastiquement vers leur position de rapprochement, notamment par l'élasticité naturelle d'un matériau isolant qui enrobe l'ensemble des éléments et contribue avec ceux-ci à former l'embase;

- l'électrode a une extrémité libre non blessante, notamment en forme de calotte sphérique;

- un textile à mailles assez lâches est fixé à l'embase et s'étend autour de l'électrode.

L'invention sera mieux comprise par la description détaillée ci-après faite en référence au dessin annexé. Bien entendu, la description et le dessin ne sont donnés qu'à titre d'exemple indicatif et non limitatif.

La figure 1 est une vue schématique en coupe d'un dispositif conforme à l'invention selon un premier mode de réalisation.

La figure 2 est une vue schématique illustrant la mise en place d'un dispositif conforme à l'invention.

La figure 3 est une vue schématique partielle en coupe montrant un deuxième mode de réalisation de l'invention.

La figure 4 est une vue schématique en coupe du même dispositif que celui représenté sur la figure 3.

La figure 5 est une vue schématique montrant un dispositif conforme à l'invention fixé à l'extérieur d'un coeur.

La figure 6 est une vue schématique en coupe d'un troisième mode de réalisation de l'invention.

Les figures 7 à 9 illustrent une variante de ce troisième mode de réalisation.

Les figures 10 et 11 sont des vues schématiques en coupe d'un quatrième mode de réalisation de l'invention.

La figure 12 est une vue schématique dans le plan indiqué par la ligne XII-XII de la figure 10.

En se reportant au dessin, on voit qu'un dispositif conforme à l'invention comprend une électrode conductrice 1 assujettie à une embase 2 qui est réalisée en un matériau isolant afin d'emprisonner à la fois une partie de l'électrode 1 pour la retenir et les connexions électriques avec un fil conducteur 3 par ailleurs placé dans une gaine isolante 4.

Un textile 5 à mailles assez lâches est fixé à l'embase 2 et s'étend autour de l'électrode 1.

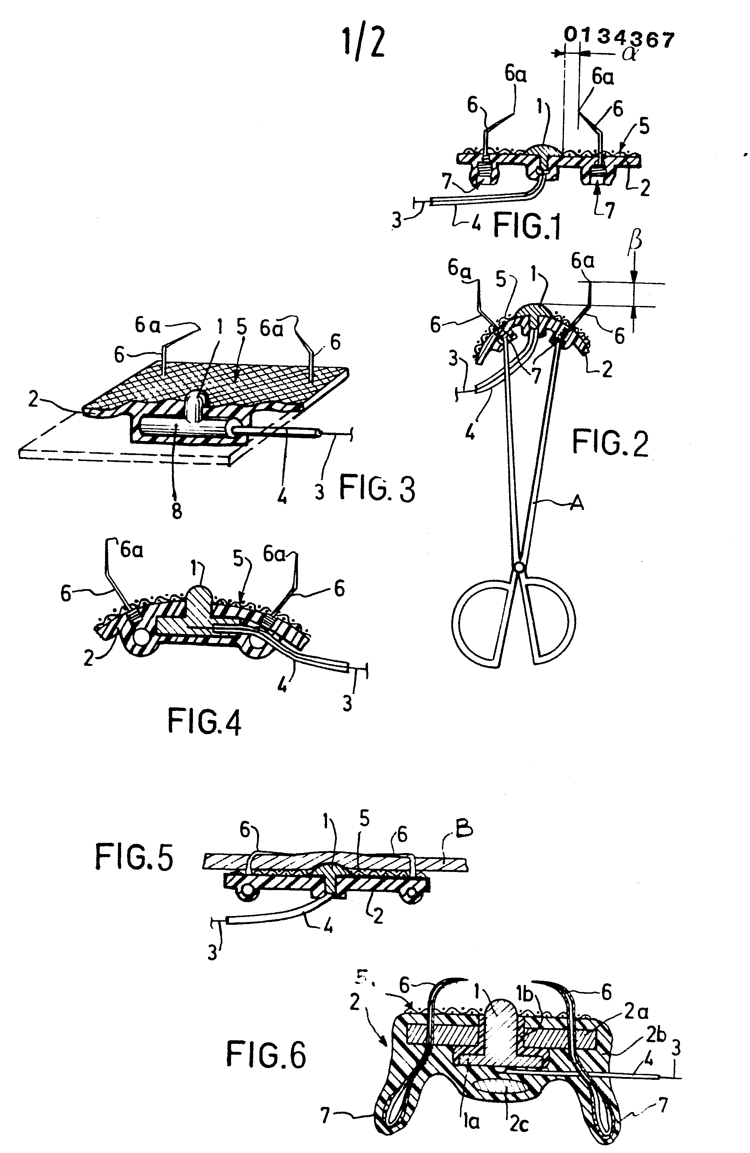

La fixation du dispositif de la figure 1 contre la paroi cardiaque d'un patient au moyen de crochets provoque la formation de fibrine par le sang, fibrine qui s'enchevêtre avec les fils constituant le textile 5, de sorte qu'après un certain temps, le dispositif est maintenu en place par la fibrine naturelle. Mais il s'agit d'une fixation secondaire car elle n'intervient qu'après une fixation primaire réalisée par des crochets 6, comme on va le décrire maintenant.

Les crochets 6 sont situés assez loin de l'électrode 1 placée au centre de l'embase 2 pour que les extrémités 6a des crochets 6, rabattues lorsque les crochets 6 sont en position active, se trouvent hors du périmètre de l'électrode 1, dans un même plan ou au-dessus d'elle, bien qu'ils soient "convergents", c'est-à- dire repliés les uns vers les autres, au moins deux à deux, un peu à la manière des griffes d'une patte d'animal.

Avec ces dispositions, non seulement on empêche tout contact entre l'électrode 1 et les crochets 6 mais, même, on repousse la formation de fibrine hors de la zone où se trouve l'électrode 1, de sorte que cette fibrine dont la formation est provoquée par la pénétration des crochets 6 ne vient pas gêner, par sa présence, la bonne conductivité de la paroi cardiaque équipée.

Sur la figure 1 on voit qu'entre l'extrémité 6a des crochets 6 et la périphérie de l'électrode 1, subsiste un espace $\alpha$ mesuré depuis la périphérie de l'électrode 1 jusqu'à l'aplomb des extrémités 6a.

Cet espace $\alpha$ est le même avant et après fixation car celle-ci ne s'obtient pas par déformation des crochets 6 à la manière d'une agrafe mais par déformation d'une embase 2 qui est exécutée en un matériau élastique.

Sur la figure 2 on voit comment cela peut être obtenu.

Sur sa face opposée à celle qui porte l'électrode 1, l'embase 2 est munie de deux appuis 7 constitués ici par des petits cylindres rigides placés en inserts dans le matériau qui constitue l'embase 2.

De la sorte, on peut engager les extrémités d'une pince A de tout type connu dans les logements formés par les inserts et cintrer l'embase 2, comme cela est représenté sur la figure 2 en utilisant les appuis 7.

Ce cintrage a pour effet d'orienter les crochets 6 de telle manière que leurs extrémités 6a se présentent face à la paroi cardiaque et il suffit au chirurgien de les enfoncer sans autre mouvement latéral, rotatoire ou complexe que celui qui consiste à pousser droit devant soi. En relachant l'effort exercé sur la pince A, l'embase 2 reprend sa forme primitive grâce à son élasticité et les crochets 6 reprennent la position qu'ils ont sur la figure 1 qui est celle de la fixation effective.

On obtient ainsi le maintien convenable de l'électrode 1 contre la paroi extérieure du coeur grâce à l'effet combiné de l'élasticité de l'embase 2 et de la forme des crochets 6, effet qui tend à solliciter la paroi cardiaque et l'électrode 1, l'une vers l'autre.

Cette électrode 1 peut alors jouer le rôle qui est le sien, indépendamment du rôle de fixation qui appartient à titre primaire aux crochets 6 et à titre secondaire à la fibrine enchevêtrée dans le textile 5.

L'électrode 1 a de préférence la forme d'une calotte sphérique pour éviter toute blessure de la paroi cardiaque et assurer le contact selon toute sa surface car l'effet de pincement de la paroi entre les crochets 6 et l'embase 2 provoque une petite déformation de la paroi cardiaque comme on le voit plus précisément sur la figure 5.

On voit sur la figure 2, qu'en position de cintrage de l'embase 2, les crochets 6 dépassent le sommet de l'électrode 1 d'une distance $\beta$ grâce à laquelle ils atteignent la paroi cardiaque et y pénétrent avant que l'électrode 1 arrive à son tour au contact de ladite paroi cardiaque.

On atteint ainsi le but poursuivi par l'invention en établissant convenablement les dimensions relatives de l'électrode 1 et des crochets 6.

Mais, dans la pratique, cette solution n'est pas toujours possible compte tenu des impératifs dimensionnels liés à la physiologie. Il faut alors s'opposer à la protrusion de l'électrode 1, soit en l'immobilisant, soit même en la faisant reculer tandis que les crochets 6 sont redressés vers l'avant.

Sur les figures 3 et 4 on a représenté un mode de réalisation selon lequel on immobilise l'électrode 1.

Pour cela, on prévoit que sur sa face opposée à celle qui porte l'électrode 1, l'embase 2 comprend un élément rigide 8 situé à l'aplomb de l'électrode 1 et placé perpendiculairement au sens selon lequel on doit cintrer l'embase 2.

Ici, on a prévu que l'élément 8 constitue la branche d'un T dont le jambage constitue l'électrode 1, le tout étant/noyé dans une matière synthétique qui constitue l'embase 2, à l'exception de l'électrode 1 qui, bien évidemment, doit se situer en avant du plan supérieur de l'embase 2.

En se reportant à la figure 4, on voit que l'embase 2 est munie de deux appuis 7 constitués ici par des anneaux noyés dans la matière constitutive de l'embase 2 mais débouchant à l'extérieur. La présence de ces appuis 7 donne automatiquement le sens dans lequel on doit cintrer l'embase 2 pour donner aux crochets 6 l'orientation nécessaire à leur pénétration rectiligne. L'organe 8 est situé entre ces appuis 7 de telle manière qu'il s'oppose à la flexion de l'embase 2 dans sa partie centrale c'est-à-dire entre les appuis 7 et les crochets 6, ce qui empêche la protrusion de l'électrode 1 sans pour autant gêner la mise en position des crochets 6.

Sur la figure 5 on voit un dispositif conforme aux figures 1 et 2 mis en place sur la paroi B d'un coeur.

En l'occurrence, on a représenté cette paroi B comme ayant une épaisseur très faible, ce qui correspond à la paroi de l'oreillette droite et l'on voit que si les crochets 6 ont complètement traversé cette paroi, l'électrode 1 n'en est pas moins parfaitement placée au contact extérieur de la paroi B malgré la minceur de cette dernière.

La fibrine sera formée dans la zone de perforation des crochets 6 c'est-à-dire loin du centre où se situe l'électrode 1, de sorte que les caractéristiques de conductivité électrique ne seront pas modifiées.

Le fonctionnement de l'électrode 1 ne varie donc pas et comporte comme cela est connu, la double fonction d'amenée de courant vers le coeur et de transmission depuis le coeur des caractéristiques auxquelles le stimulateur est sensible.

Comme on l'a vu, le dispositif conforme à l'invention permet une fixation très simple qui ne nécessite pas de développer un effort important ou de réaliser des gestes difficiles. Le chirurgien peut donc emprunter une voie d'approche simple, peu étendue et non dandereuse.

Ainsi, par exemple, il est possible d'agir par voie épigastrique c'est-à-dire en passant à travers le diaphragme, sous le sternum, sans ouvrir les cavités abdominale ou thoracique, cette voie permettant néanmoins de placer deux dispositifs, l'un sur l'oreillette droite et l'autre sur le ventricule droit.

L'invention permet donc la mise en place de stimulateurs à deux électrodes dont l'intérêt est majeur, du fait qu'ils permettent un fonctionnement du coeur conforme à la physiologie.

Compte tenu de la possibilité d'une fixation secondaire au moyen du textile 5 dans lequel la fibrine s'enchevêtre, il est possible d'utiliser une fixation primaire temporaire, avec des crochets en réalisant ceux-ci dans une matière résorbable dans un temps bien entendu supérieur à celui qui est nécessaire à la formation de fibrine.

La mise en place d'un dispositif conforme à l'invention ne nécessite aucun geste autre que celui de l'approche rectiligne.

Pour déterminer l'emplacement le plus favorable à l'implantation de l'électrode 1, on peut utiliser un détecteur à l'extrémité d'un support de tout type connu et relié par un fil à un appareil de mesure.

On peut déplacer le détecteur le long de la paroi du coeur, de place en place, jusqu'à ce que l'instrument de mesure révèle le meilleur emplacement. A ce moment, grâce à sa facilité de mise en place, il suffit de substituer le support de détecteur par un support du dispositif conforme à l'invention, pour fixer ce dispositif à l'endroit voulu.

A cet égard, on peut utiliser un tube souple plus ou moins long dans l'embouchure duquel le dispositif est placé, l'embase 2 étant cintrée, et de placer un mandrin dans le tube de telle sorte que la mise en place du dispositif se fait en maintenant ce dernier par le mandrin et en effectuant un petit recul du tube pour libérer l'embase 2.

L'embase 2 peut avoit une structure simple, comme on vient de la décrire ou composite ainsi que cela est représenté sur les figures 6 à 12 .

On voit sur la figure 6 que l'embase 2 comprend une âme 2a (en métal ou en matière synthétique) enrobée d'une matière élastique, neutre et électriquement isolante 2b , notamment à base de silicone ou de polyuréthane.

L'âme 2a, elle-même élastiquement déformable, est solidaire de la fixation primaire (c'est-à-dire des crochets 6) et des appuis 7 par lesquels on peut déformer l'embase 2 lors de la mise en place du dispositif.

L'électrode 1 est munie d'un épaulement circulaire 1a pouvant buter autour d'un trou central 2c de l'âme 2a par lequel l'électrode 1 est engagée après revêtement d'un isolant 1b qui laisse son extrémité dégagée pour le contact avec la paroi cardiaque.

L'enrobage 2b laisse passer l'extrémité de l'électrode 1 et les crochets 6 et se prolonge sur la gaine 4 du fil conducteur 3.

La partie de l'enrobage 2b située sous l'épaulement 1a de l'électrode 1, forme un coussinet élastique 2d qui tend à pousser l'électrode 1 vers le coeur lorsque l'embase 2 est fixée par les crochets 6.

On peut encore accroître cet effet en prévoyant un insert 2e en toute matière voulue.

Au moment de la mise en place, au contraire, le coussinet 2d et l'insert 2e agissent comme l'organe 8 et empêchent la formation d'un dos d'âne. Mais, en outre, le rapprochement des appuis 7 a pour conséquence l'abaissement des bords de l'âme 2a et une sorte de pincement central qui a pour effet de pousser l'épaulement 1a vers le bas, c'est-à-dire d'empêcher la protrusion de l'électrode 1.

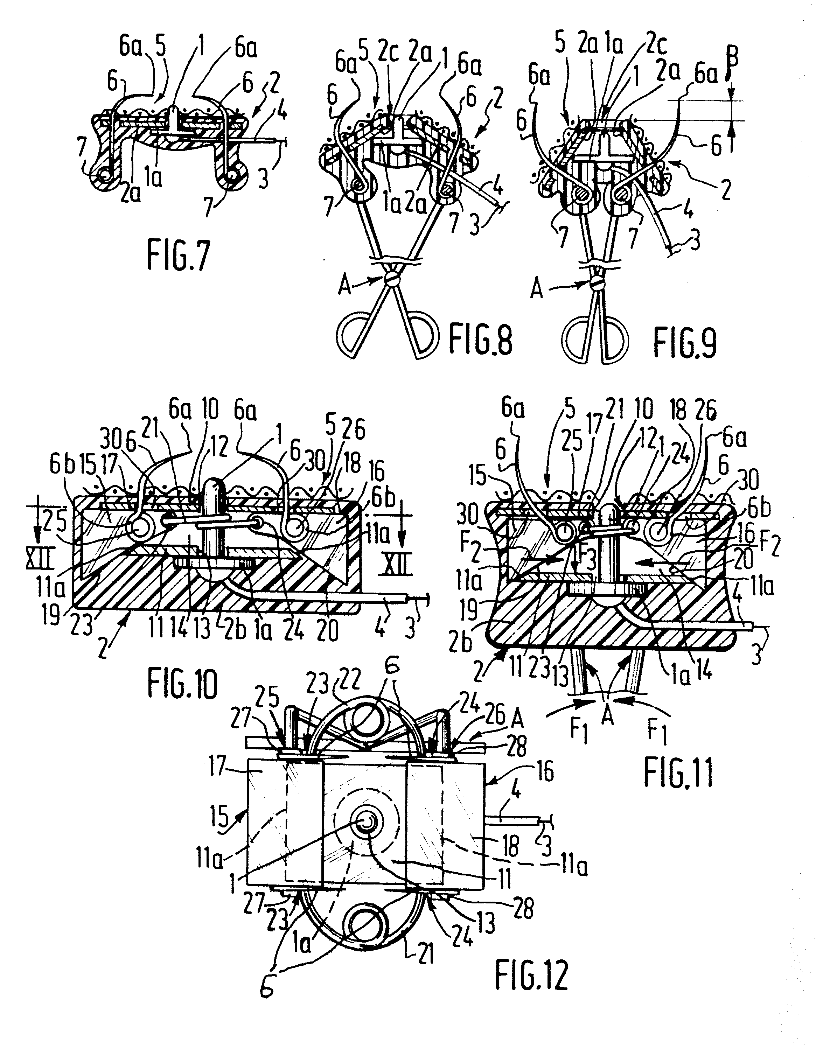

Ce phénomène sera décrit en détail plus loin, en regard des figures 7 à 9.

Il faut souligner que le matériau d'enrobage, bien connu en lui-même par l'homme de métier, est d'une extrême souplesse et se prête facilement, par étirement, contraction et/ou déformation à toutes les sollicitations nécessaires. En particulier, le revêtement 1b glisse sans aucun effort dans le trou 2c car la partie supérieure 2f du matériau d'enrobage ne colle pas à lui.

L'enrobage 2b doit avoir des formes et une épaisseur qui s'opposent à l'éloignement de l'électrode 1 de la paroi cardiaque et qui favorisent l'application non agressive de cette électrode 1 sur le coeur quand l'embase 2 est fixée par les crochets 6, notamment en pouvant suivre les déformations naturelles du coeur.      **0134367**

Outre qu'elle doit être déformable pour pouvoir donner aux crochets  6 leur position d'approche et leur position de fixation, l'embase 2  doit avoir une étendue et une résistance suffisante pour recevoir le nombre de crochets voulu. Elle doit laisser l'extrémité de l'électrode  1 bien dégagée.

Il faut éviter que le courant électrique atteigne, par accident, une partie du dispositif autre que l'extrémité de l'électrode 1 . A cette fin, on prévoit avantageusement que l'isolant 1b s'étend jusque sur l'épaulement 1a , ce qui évite de transmettre le courant à l'âme 2a même si par usure ou par sollicitation mécanique de la matière d'enrobage 2b, cet épaulement vient au contact de l'âme 2a.

De même il est bon de recouvrir les crochets 6 et les appuis 7 d'un revêtement isolant.

Naturellement, ces précautions sont à prendre lorsque les pièces en cause sont faites en un matériau conducteur de l'électricité, hypothèse qui est celle retenue pour la figure 6. Si, par exemple, l'âme 2a est en un matériau isolant tel qu'une matière synthétique, la présence de l'isolant 1b est inutile.

Avec les dispositions qui viennent d'être décrites, le cintrage de l'embase 2 qui entraîne le redressement des crochets 6 est assuré mais  empêche la formation d'un dos d'âne central qui aurait pour conséquence de pousser l'élec- trode 1 vers l'avant.

L'électrode 1 est en quelque sorte bloquée par la pièce 8 (figures 3 et 4) ou par le coussinet 2d (figure 6).

En se reportant maintenant aux figures 7 à 9, on voit que l'épaulement 1a de l'électrode 1 est directement en contact avec l'âme 2a qui est, donc, supposée isolante. En outre   il   n'y a pas d'insert 2e. Mais ces différences de détail n'ont pas de conséquence sur le fonctionnement que l'on va décrire maintenant et qui s'applique aussi bien au mode  de réalisation de la figure 6 qu'à sa variante des figures 7 à 9.

Sur la figure 7, le dispositif est en position de repos avant sa mise en place.

Une pince A, du type connu comprenant des branches à bec recourbé, est utilisée pour agir  sur les appuis 7 en vue de plier l'embase 2. Sur la  figure 8 on voit  une position intermédiaire dans laquelle les crochets 6 sont redressés partiellement.

L'abaissement des bords extérieurs de l'âme 2a provoque le soulèvement de sa partie centrale où  se trouve le trou 2c . L'âme  2a se plie en formant un angle aigu qui se ferme de plus en plus au fur et à mesure que l'on rapproche les appuis 7 et, ainsi, s'exerce sur l'épaulement 1a un effort dirigé vers le bas qui oblige cet épaulement 1a et  l'électrode 1 dont il fait partie à reculer par rapport au niveau des extrémités 6a des crochets 6 qui, elles, s'élèvent quand l'électrode s'abaisse.

Sur la figure 9, on voit l'embase 2 complètement pliée et les crochets 6 redressés. Les extrémités 6a sont en avant de tout l'ensemble et, lors de l'approche, elles atteindront les premières la paroi cardiaque. Aussitôt après leur pénétration, le chirurgien relâche avec précaution les branches de la pince A et l'élasticité naturelle de l'embase 2 oblige celle-ci à reprendre sa forme stable (figure 7).

Les crochets 6 se rabattent l'un vers l'autre (en convergence vraie si les crochets 6 sont répartis angulairement, ou deux à deux en pince s'ils sont placés face à face), le centre de l'embase 2 s'abaisse, l'électrode 1 se soulève et vient au contact de la paroi cardiaque, le tissu 5 étant intercalé entre cette paroi et le dessus de l'embase 2.

On voit qu'ici, contrairement au mode de réalisation des figures 3 et 4, on ne se contente pas d'immobiliser l'électrode 1, on provoque délibérément son recul.

Bien entendu, les dessins sont des exemples schématiques et, dans la pratique , on adopte les dimensions les plus propices grâce auxquelles, notamment, la distance $\beta$ est la plus grande possible. On note qu'ici cette distance est mesurée à partir de la partie centrale de l'âme 2a et non à partir du sommet de l'électrode 1 car celui-ci est situé en retrait et la partie centrale de l'âme 2a constitue la partie la plus proéminente du dispositif.

En se reportant maintenant aux figures 10 à 12, on voit un autre mode de réalisation selon lequel les crochets 6 sont montés mobiles par rapport à l'embase 2.

Le redressement des crochets 6 n'est donc plus une conséquence de la déformation de l'embase 2. Celle-ci doit toujours être élastiquement déformable mais l'effort que le chirurgien exerce par une pince A s'applique au déplacement direct des crochets, l'élasticité de l'embase 2 agissant en retour pour remettre le dispositif en position stable.

Pour cela, les moyens aptes à s'opposer à la protrusion de l'électrode 1 tout en permettant le redressement des crochets 6, sont constitués par deux éléments plans 10 et 11 tels que des plaquettes rigides, indéformables avec les efforts raisonnables ici en cause, en matériau isolant.

Les plaquettes 10 et 11 sont superposées et traversées chacune par un trou central respectivement 12 et 13. Ces trous sont alignés et reçoivent l'électrode 1 engagée par le trou 13 jusqu'à ce que son épaulement 1a bute contre la plaquette 11.

Les deux plaquettes 10 et 11 sont séparées par un espace 14 en regard duquel sont placés latéralement des écarteurs constitués ici
par deux coins mobiles 15 et 16. Leurs faces supérieures 17 et 18 sont
parallèles à la plaquette 10 et leurs faces situées en vis-à-vis 19 et 20
sont obliques pour former des rampes qui sont en contact avec les bords
latéraux 11a de la plaquette 11. Si cette dernière a une épaisseur substantielle, il peut s'avérer utile de prévoir ces bords 11a taillés en
biseau, comme cela est représenté, pour qu'ils soient parallèles aux
rampes 19 et 20.

Les coins 15 et 16 sont réunis par des ressorts 21 et 22 cintrés en
épingle et dont les extrémités sont engagées dans des trous 23 et 24
afin que les coins 15 et 16 soient constamment sollicités vers leur position d'écartement représentée sur la figure 10.

Sur les côtés des coins 15 et 16, se trouvent des trous 25 et 26
pour engager les becs des branches de la pince A. En outre, des moyens
sont prévus pour la fixation des crochets 6. Ces moyens peuvent consister
simplement à noyer l'extrémité des crochets dans la masse des coins 15 et
16 quand ceux-ci sont moulés en une matière synthétique. Ici, on a
représenté une solution parmi d'innombrables autres et qui consiste
à prévoir une boucle 6b sur chacun des crochets 6, puis à engager
chacune de ces boucles 6b sur un relief 27 (coin 15) ou 28 (coin 16).
Les trous 25 et 26 sont prévus au centre de ces reliefs 27 et 28.

Les crochets 6 passent par des trous 30 prévus dans la plaquette
10 selon un diamètre grand relativement à celui des crochets 6.

Enfin, toutes ces pièces à l'exception des crochets 6 proprement
dits, de l'électrode 1 et du fil 3 dans sa gaine 4, sont noyées dans une
matière élastique, souple et isolante 2b pour former un ensemble constituant l'embase 2. Toutefois, on peut sans grand inconvénient laisser les
ressorts 21 et 22 dépasser hors de la matière d'enrobage 2b. L'ensemble
est terminé par apposition du tissu 5.

Le fonctionnement de ce dispositif est le suivant:
Au repos, c'est-à-dire avant ou après mise en place, le dispositif est
dans la position de la figure 10. Pour la mise en place, on engage les
becs des branches de la pince A dans un trou 25 du coin 15 et un trou 26
du coin 16, sur un même côté, ces trous 25 et 26 débouchant à l'extérieur

En fermant la pince A selon les flèches F1 (Figure 11), on déplace les coins 15 et 16 en les approchant l'un vers l'autre selon les

**0134367**

flèches F2 (figure 11), contre l'action antagoniste des ressorts 21 et 22. Les rampes 19 et 20 poussent les bords 11a de la plaquette 11 selon une direction résultante perpendiculaire au plan de la plaquette 11 indiquée par la flèche F3 sur la figure 11, ce qui a pour conséquence d'écarter les plaquettes 10 et 11 en augmentant la hauteur de l'espace 14.

Ce faisant, la plaquette 11 entraîne l'électrode 1 par son épaulement 1a et la fait pénétrer dans le contour de l'embase 2, le sommet de l'électrode 1 reculant, éventuellement jusqu'à s'effacer pour ne plus faire aucune saillie extérieure.

Pendant ce mouvement, les crochets 6 liés aux coins 15 et 16 se redressent pour passer de leur position stable de la figure 10 à leur position de mise en place de la figure 11.

Si les trous 30 de la plaquette 10 ont un diamètre relativement petit, les crochets 6 doivent fléchir et se cintrer, mais cela est facile à obtenir dans la pratique car on connait bien les métaux inoxydables qui ont les propriétés voulues pour présenter à la fois la malléabilité nécessaire à cette flexion et la nervosité indispensable à leur fonction de fixation sûre.

Si les trous de la plaquette 10 ont un diamètre relativement grand (considéré dans le plan du dessin), les crochets 6 n'ont pas à fléchir. En revanche, ils doivent obligatoirement se redresser et cela est obtenu du fait que le rapprochement des coins 15 et 16 les fait buter contre le bord des trous 30 (figure 11).

L'écartement des plaquettes 10 et 11 provoque l'allongement et le rétrécissement de l'embase 2, comme on peut l'observer schématiquement par comparaison des figures 10 et 11. Dans cette position, le dispositif est engagé dans la voie d'accès préparée par le chirurgien, puis il est appliqué directement, sans geste supplémentaire. Les crochets 6 sont en avant de l'embase 2 et atteignent de front la paroi cardiaque. Dès qu'ils sont enfoncés, le chirurgien relâche avec précaution les branches de la pince A. Les ressorts 21 et 22 repoussent les coins 15 et 16 qui provoquent le rabattement des crochets 6 butant contre le bord des trous 30, à l'opposé du cas précédemment décrit. L'élasticité naturelle de la matière 2b soulève la plaquette 11 et l'épaulement 1a de l'électrode 1.

L'ensemble reprend ainsi sa position stable de la figure 10.

L'électrode 1 est en appui contre l'extérieur de la paroi cardiaque et les crochets 6 forment un contre-appui contribuant à l'application constante de l'électrode 1 contre le coeur. Le tissu 5 est prêt à recueillir la fibrine pour former une fixation secondaire.

L'extrémité, ou sommet, de l'électrode 1 en forme de calotte sphérique assure un contact efficace, doux et non blessant.

L'appui de l'épaulement 1a par sa face arrière contre la matière 2b qui est élastique et très souple permet à l'électrode 1 de suivre les mouvements naturels du coeur en reculant puis en avançant élastiquement, au gré du fonctionnement cardiaque, sans jamais interrompre le contact et sans provoquer la moindre lésion.

Les écarteurs peuvent être réalisés par des moyens mécaniques connus en soi et différents des coins 15 et 16 : excentriques, cames, genouillères, etc.

16

**0134367**

1 - Dispositif de stimulation cardiaque, du type comprenant une électrode conductrice (1) associée à une embase (2) élastiquement déformable devant être mise en place sur le coeur (B) d'un patient et être fixée à l'extérieur dudit coeur (B) par des crochets (6) , caractérisé en ce que les extrémités libres (6a) des crochets 6 sont situées au-delà de la partie la plus proéminente du dispositif et en particulier du sommet de l'électrode (1), selon une distance (P) substantielle lorsqu'ils sont en position de redressement due à la déformation élastique de l'embase (2).

2 - Dispositif selon la revendication 1, caractérisé en ce qu'il comprend des moyens aptes à s'opposer à la protrusion de l'électrode (1) tout en permettant le redressement des crochets (6).

3 - Dispositif selon la revendication 2, caractérisé en ce que l'embase (2) devant être déformée élastiquement, par cintrage, lesdits moyens sont constitués par un élément (8) rigide dans le sens perpendiculaire à celui selon lequel on doit cintrer l'embase (2) lors de sa mise en place.

4 - Dispositif selon la revendication 3, caractérisé en ce que l'élément est une pièce conductrice (8) en forme de T dont la branche est située dans l'embase (2) perpendiculairement au sens selon lequel on doit cintrer celle-ci et dont le jambage constitue l'électrode (1).

5 - Dispositif selon la revendication 2, caractérisé en ce que lesdits moyens sont constitués par un élément substantiellement plan (2a) qui est rigide mais pliable en son milieu et qui est traversé d'un trou central (2c) par lequel l'électrode (1) est engagée librement, cet élément étant relié aux crochets (6) et à des appuis (7) destinés à recevoir un effort pour le pliage dudit élément et, ipso facto, le cintrage de l'embase (2).

6 - Dispositif selon la revendication 5, caractérisé en ce que l'électrode (1) est munie d'un épaulement (1a) disposé sous l'élément (2a) par rapport à l'extrémité libre de ladite électrode (1) et pouvant buter autour du trou central (2c) de l'élément (2a)

7 - Dispositif selon la revendication 5, caractérisé en ce que l'embase (2) étant réalisée en matériau élastique isolant emprisonnant l'électrode (1), les crochets (6) et les appuis (7), une partie ue ce matériau d'en obage (2b) située sous l'électrode (1) par rapport à son extrémité libre, forme un coussinet élastique (2d) contenant éventuellement un insert (2e).

8 - Dispositif selon la revendication 2 caractérisé en ce que les crochets (6) étant mobiles par rapport à l'embase (2), lesdits moyens sont constitués par deux éléments superposés (10 et 11) substantiellement plans, rigides, indéformables et traversés chacun par un trou central (12-13) par lesquels l'électrode (1) est engagée librement et bute, par un épaulement (1a) dont elle est munie, contre l'élément plan (11) le plus éloigné des extrémités libres des crochets (6), tandis que des écarteurs mobiles (15 et 16) sont disposés latéralement en regard d'un espace (14) qui sépare les deux éléments (10 et 11) et sont reliés aux crochets (6) et à des appuis (25-26) destinés à recevoir un effort pour le rapprochement des écarteurs (15 et 16), l'écartement corrélatif des éléments (10 et 11) ainsi que le retrait de l'électrode (1) et le redressement des crochets (6).

9 - Dispositif selon la revendication 8, caractérisé en ce que les écarteurs (15 et 16) sont des coins sollicités élastiquement vers leur position d'écartement, notamment au moyen d'un ressort (21-22) tandis que les éléments (10 et 11) sont sollicités élastiquement vers leur position de rapprochement, notamment par l'élasticité naturelle d'un matériau isolant (2b) qui enrobe l'ensemble des éléments et contribue avec ceux-ci à former l'embase (2).

10 - Dispositif selon la revendication 1 caractérisé en ce que l'électrode (1) a une extrémité libre non blessante, notamment en forme de calotte sphérique.

11-Dispositif selon la revendication 1, caractérisé en ce qu'un textile (5) à mailles assez lâches est fixé à l'embase (2) et s'étend autour de l'électrode (1).

0134367

**FIG.1**

**FIG.2**

**FIG.3**

**FIG.4**

**FIG.5**

**FIG.6**

0134367

FIG.7

FIG.8

FIG.9

FIG.10

FIG.11

FIG.12

## 0134367

Office européen
des brevets

**RAPPORT DE RECHERCHE EUROPEENNE**

Numéro de la demande

EP 83 40 1656

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl. ³) |
|---|---|---|---|
| X,D | FR-A-2 297 641 (CORDIS)<br>* Page 4, lignes 23-37; figure 7 * | 1-4 | A 61 N 1/04 |
| A,D | FR-A-2 372 636 (DE PEDRO)<br>* Page 2, lignes 32-38; page 3, lignes 20-24; figure 3 * | 1-4 | |
| A | FR-A-2 466 255 (PETIT)<br>* Page 2, lignes 16-26 * | 1,11 | |
| A | US-A-3 976 082 (SCHMITT)<br>* Colonne 2, ligne 41 - colonne 3, ligne 20 * | 8,10 | |
| E | FR-A-2 525 110 (ALFANDARI)<br>* En entier * | 1-7,10,11 | |

DOMAINES TECHNIQUES
RECHERCHES (Int. Cl. ³)

A 61 N

Le present rapport de recherche a été établi pour toutes les revendications

| Lieu de la recherche | Date d'achèvement de la recherche | Examinateur |
|---|---|---|
| LA HAYE | 05-04-1984 | SIMON J.J.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82